# FASCICULE DE BREVET EUROPEEN

(11) **EP 0 799 833 B1**
(45) Date de publication et mention de la délivrance du brevet: **29.05.2002**
(21) Numéro de dépôt: 96402821.1
(22) Date de dépôt: 19.12.1996
(51) Int. Cl.: C07H 17/00, C07H 17/08, A61K 31/70

(54) **Nouveaux dérivés de l'érythromycine, leur procédé de préparation et leur application comme médicaments**
Erythromycin-Derivate, Verfahren zu ihrer Herstellung und ihre Verwendung als Arzneimittel
Erythromycin derivatives, their production and their use as medicaments

(30) Priorité: 22.12.1995 FR 9515322
(43) Date de publication de la demande: 08.10.1997
(73) Titulaire: Aventis Pharma S.A., 92160 Antony (FR)
(72) Inventeur: Agouridas, Constantin, 94130 Nogent Sur Marne (FR); Bretin, François, 77330 Ozoir La Ferriere (FR); Chantot, Jean-François, 94130 Nogent Sur Marne (FR)

(56) Documents cités:
- EP-A- 0 487 411
- EP-A- 0 596 802
- EP-A- 0 676 409
- EP-A- 0 680 967

## Description

La présente invention concerne de nouveaux dérivés de l'érythromycine leur procédé de préparation et leur application comme médicaments.

Les demandes européennes EP-A-0680967 et 0676409 décrivent des dérivés de l'érythromycin apparentés à ceux de la présente demande, et possédant des propriétés antibactériennes, comportant en position 2 un radical méthyle.

La présente demande a pour objet des composés comportant en position 2 à la fois un radical méthyle et un atome d'halogène. Par rapport aux composés non halogénés en 2, ils présentent des propriétés antibactériennes intéressantes et notamment un gain d'activité notable sur cocci érythromycine résistants de façon constitutive et, pour certains, sur entérocoques et Haemophilus.

L'invention a pour objet les composés de formule (I) : 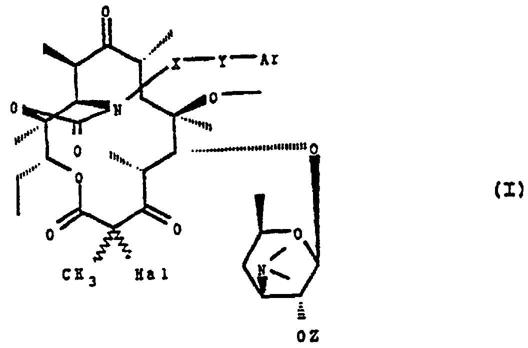 dans lesquels
- X représente un radical (NH)a, CH₂ ou SO₂ ou un atome d'oxygène, a représente le nombre 0 ou 1,
- Y représente un radical (CH₂)m-(CH=CH)n-(CH₂)o avec m+n+o ≤ 8, n = 0 ou 1,
- Ar représente un radical aryle ou hétéroaryle, éventuellement substitué
- Hal représente un atome d'halogène
et Z représente un atome d'hydrogène ou le reste d'un acide ainsi que leurs sels d'addition avec les acides.

Le radical aryle peut être un radical phényle ou naphtyle.

Le radical aryle peut être également un radical hétérocyclique substitué ou non comme le radical thiényle, furyle, pyrolyle, thiazolyle, oxazolyle, imidazolyle, thiadiazolyle, pyrazolyle ou isopyrazolyle, un radical pyridyle, pyrimidyle, pyridazinyle ou pyrazinyle, ou encore un radical indolyle benzofurannyle, benzothiazyle ou quinoléinyle.

Ces radicaux aryles peuvent comporter un ou plusieurs groupements choisis dans le groupe constitué par les radicaux hydroxyle, les atomes d'halogène, les radicaux NO₂, les radicaux C≡N, les radicaux alkyle, alkényle ou alkynyle, O-alkyle,O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 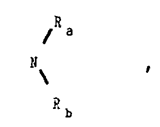 Rₐ et R_{b} identiques ou différents, représentant un atome d'hydrogène ou un radical alkyle renfermant jusqu'à 12 atomes de carbone, le radical 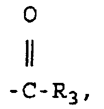 R₃ représentant un radical alkyle renfermant jusqu'à 12 atomes de carbone, ou un radical aryle ou hétéroaryle éventuellement substitué, les radicaux aryle, O-aryle ou S-aryle carboxyliques ou aryle, O-aryle ou S-aryle hétérocycliques à 5 ou 6 chaînons comportant un ou plusieurs hétéro-atomes, éventuellement substitués par un ou plusieurs des substituants mentionés ci-dessous.
Comme hétérocycle préféré, on peut citer entre autres 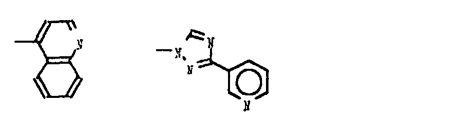 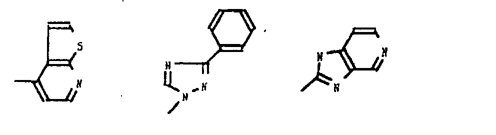 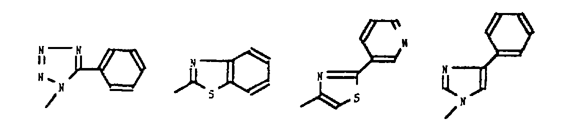 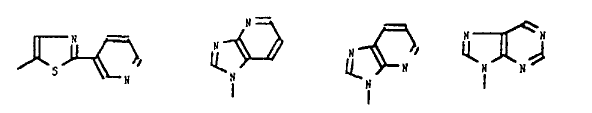 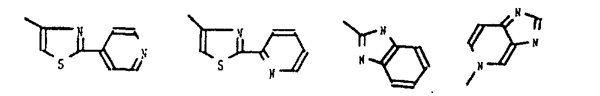 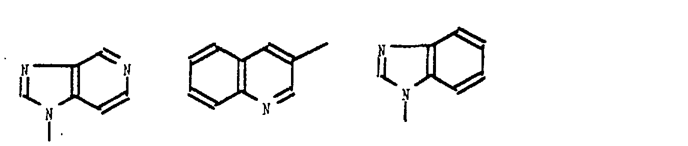 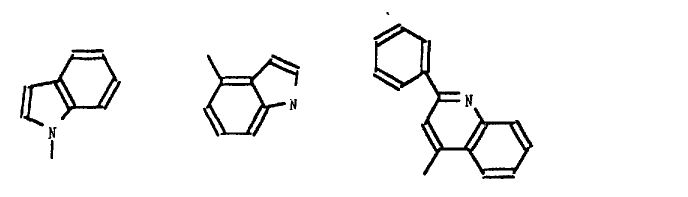 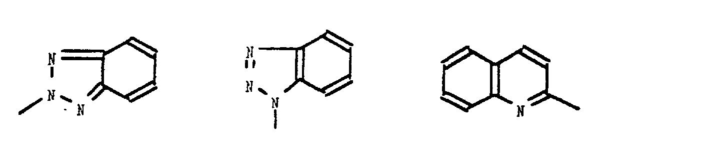 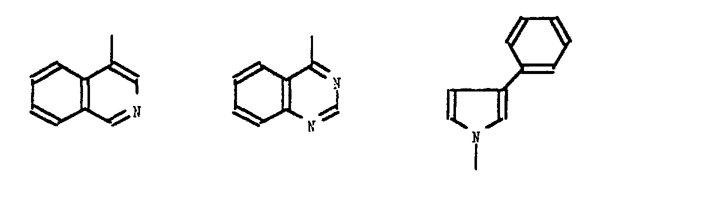 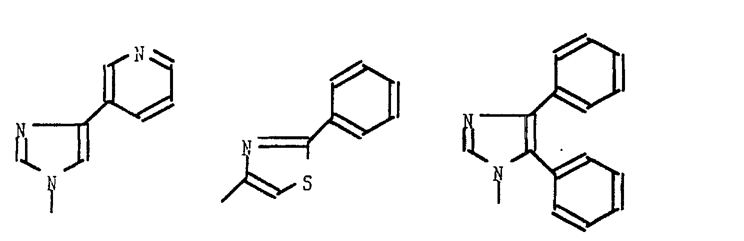 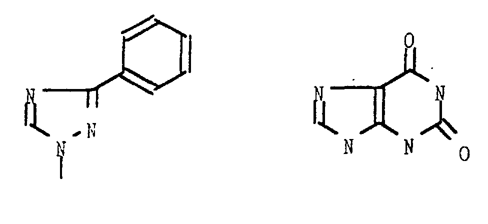 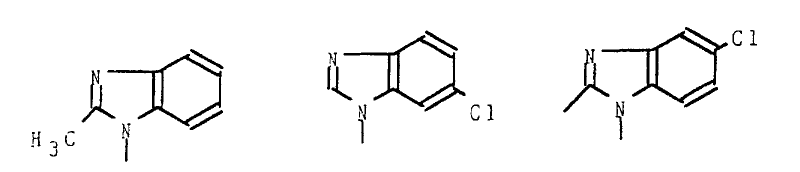 et les radicaux hétérocycliques envisagés dans les demandes de brevets européens 487411, 596802, 676409 et 680967. Ces radicaux hétérocycliques préférés pouvent être substitués par un ou plusieurs groupements fonctionnels.
Hal représente de préférence un atome de fluor, de chlore ou de brome.

Parmi les sels d'addition avec les acides, on peut citer les sels formés avec les acides acétique, propionique, trifluoroacétique, mallique, tartrique, méthanesulfonique, benzènesulfonique, p-toluènesulfonique, et spécialement les acides stéariques, éthylsuccinique ou laurylsulfonique.

L'invention a plus spécialement pour objet les composés de formule (I) dans lesquels Z représente un atome d'hydrogène, ceux dans lesquels X représente un radical (NH)a avec a = 0 ou 1, ceux dans lesquels Hal représente un atome de fluor, et ceux dans lesquels Y représente le radical (CH₂)₃, (CH₂)₄ ou (CH₂)₅.

Le radical aryle est de préférence un radical arylhétérocyclique. Parmi les composés de l'invention on peut citer les composés dans lesquels Ar représente un radical 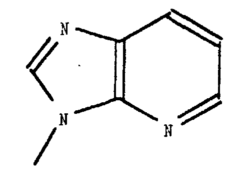 ou encore les composés de formule (I) dans lesquels Ar représente un radical 4-quinoléinyle éventuellement mono ou polysubstitué sur l'un et/ou l'autre des 2 cycles de la quinoléine et tout spécialement les composés dans lesquels Ar représente un radical 4-quinoléinyle non substitué.

Parmi les composés préférés de l'invention, on peut citer le composé de formule (I) dont le nom suit :
- 11,12-didéoxy 3-de((2,6-didéoxy-3-C-méthyl-3-O-méthyl-alpha-L-ribohexopyranosyl) oxy)-2-fluoro-6-O-méthyl-3-oxo-12,11-(oxycarbonyl((4-(3H-imidazo(4,5-b) pyridin-3-yl) butyl) imino)) érythromycine A.

Les produits de formule générale (I) possèdent une très bonne activité antibiotique sur les bactéries grain ^{⊕} telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples 1 ou 2 et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1.

L'invention a aussi pour objet un procédé de préparation des composés de formule (I) caractérisé en ce que l'on soumet un composé de formule (II) : 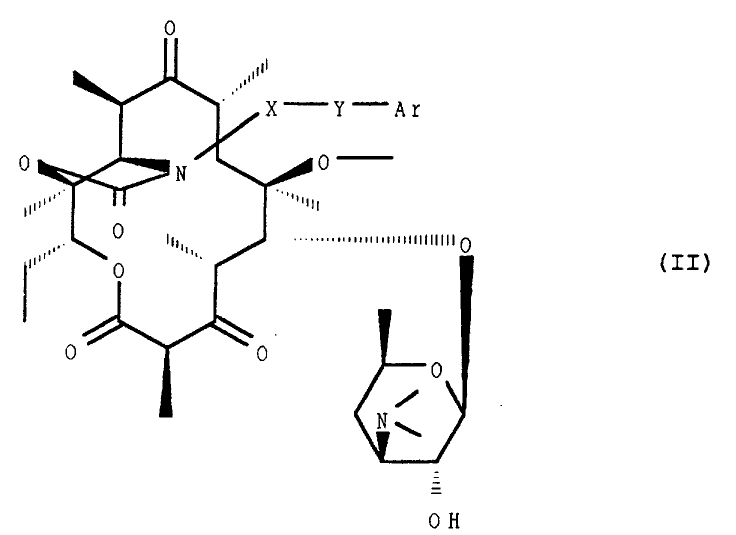 dans lesquels X, Y et Ar conservent leur signification précédente, à l'action d'un composé de formule (III) : 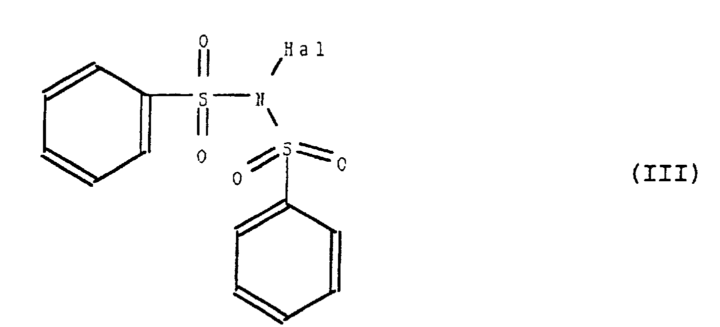 dans laquelle Hal conserve sa signification précédente, pour obtenir le composé de formule (I) correspondant que l'on soumet si désiré à l'action d'un acide pour en former le sel.

Les composés de formule (II) utilisés comme produit de départ sont décrits et revendiqués dans les demandes de brevets européens 0487411, 596802, 676409 et 680967.

Les produits de formule (III) sont des produits commerciaux.

Dans un mode de réalisation préféré du procédé de l'invention, la réaction entre le composé de formule (II) et le composé de formule (III) a lieu en présence d'une base comme l'hydrure de sodium ou la triéthylamine ou un carbonate ou un carbonate acide de sodium ou de potassium.

Les exemples suivants illustrent l'invention sans toutefois la limiter.

### EXEMPLE 1 : 11,12-didéoxy 3-de((2,6-didéoxy-3-C-méthyl-3-O-méthyl-alpha-L-ribohexopyranosyl) oxy)-2-fluoro-6-O-méthyl-3-oxo-12,11-(oxycarbonyl((4-(3H-imidazo(4,5-b) pyridin-3-yl) butyl) imino)) érythromycine (isomère A).

On ajoute à 0°C 49 mg d'hydrure de sodium dans une solution renfermant 0,502 g de 11,12-didéoxy 3-de((2,6-didéoxy-3-C-méthyl-3-O-méthyl-alpha-L-ribohexopyranosyl) oxy)-6-O-méthyl-3-oxo-12,11-(oxycarbonyl((4-(3H-imidazo(4,5-b) pyridin-3-yl) butyl) imino)) érythromycine A et 5 ml de tétrahydrofuranne. On maintient le mélange réactionnel sous agitation à 0°C pendant 1 heure et ajoute 0,303 g de N-fluoro N-(phénylsulfonyl)benzènesulfonamide, tout en laissant remonter la température.

On maintient le mélange réactionnel sous agitation, pendant 5 heures, le verse dans un mélange eau + glace. On extrait la phase aqueuse à l'aide de chlorure de méthylène, la lave avec du chlorure de méthylène. On rassemble les phases organiques, les sèche sur sulfate de magnésium, filtre et évapore à sec. On chromatographie les produits obtenus en éluant avec le mélange chlorure de méthylène, méthanol, ammoniaque (95-5-0,4). On obtient 131,3 mg de produit recherché. F = 104-106°C.

| Analyses : | | | | |
|---|---|---|---|---|
| | C % | H % | F % | N % |
| calculé | 61,2 | 7,8 | 2,4 | 8,7 |
| trouvé | 61,4 | 8 | 2,2 | 8,4 |

### EXEMPLE 2 : 11,12-didéoxy 3-de((2,6-didéoxy-3-C-méthyl-3-O-méthyl-alpha-L-ribohexopyranosyl) oxy)-2-fluoro-6-O-méthyl-3-oxo-12,11- (oxycarbonyl (2-(3-(4-quinoléinyl) 2-propyl) hydrazono)) érythromycine A

En opérant comme à l'exemple 1 à partir de la 11,12-didéoxy 3-de((2,6-didéoxy-3-C-méthyl-3-O-méthyl-alpha-L-ribohexopyranosyl) oxy) 6-O-méthyl-3-oxo-12,11-(oxycarbonyl (2-(3-(4-quinoléinyl) 2-propyl) hydrazono)) érythromycine, on a obtenu le produit recherché.

### EXEMPLE 3 : 11,12-didéoxy 3-de((2,6-didéoxy-3-C-méthyl-3-O-méthyl-alpha-L-ribohexopyranosyl) oxy)-2-fluoro-6-O-méthyl-3-oxo-12,11- (oxycarbonyl((4- (4- (3-pyridinyl) 1H-imidazol-1-yl) butyl) imino)) érythromycine A

En opérant comme à l'exemple 1, à partir de la 11,12-didéoxy 3-de((2,6-didéoxy-3-C-méthyl-3-O-méthyl-alpha-L-ribohexopyranosyl) oxy) 6-O-méthyl-3-oxo-12,11-(oxycarbonyl-((4-(4-(3-pyridinyl) 1H-imidazol-1-yl) butyl) imino)) érythromycine, on a obtenu le produit recherché.

### EXEMPLE DE COMPOSITION PHARMACEUTIQUE

On a préparé des composés renfermant :
Produit de l'exemple 1 150 mg
Excipient q.s.p. 1 g
Détail de l'excipient : amidon, talc, stéarate de magnésium

### ETUDE PHARMACOLOGIQUE DES PRODUITS DE L'INVENTION

### Méthode des dilutions en milieu liquide

On a préparé une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus :

| Souches bactériennes à GRAM+ | |
|---|---|
| Produits | Ex. 1 |
| Staphylococcus aureus 011UC4 | 0,04 |
| Staphylococcus aureus 011G025I | 0,08 |
| Staphylococcus epidermidis 012G011I | 0,15 |
| Streptococcus pyogenes groupe A 02A1UC1 | ≤ 0,02 |
| Streptococcus agalactiae groupe B 02B1HT1 | ≤ 0,02 |
| Streptococcus faecalis groupe D 02D2UC1 | 0,02 |
| Streptococcus faecium groupe D 02D3HT1 | ≤ 0,02 |
| Streptococcus sp groupe G 02G0GR5 | 0,02 |
| Streptococcus mitis 02mitCB1 | ≤ 0,02 |
| Streptococcus mitis 02mitGR16I | ≤ 0,02 |
| Streptococcus agalactiae groupe B 02B1SJ1 | 0,08 |
| Streptococcus pneumoniae 030SJ5 | 0,02 |

De plus, le produit de l'exemple 1 a montré une activité intéressante sur les souches bactériennes à gram^{⊖} suivantes : Haemophilus Influenzae 351HT3, 351CB12, 351CA1 et 351GR6.

## Revendications

1. Les composés de formule (I) : 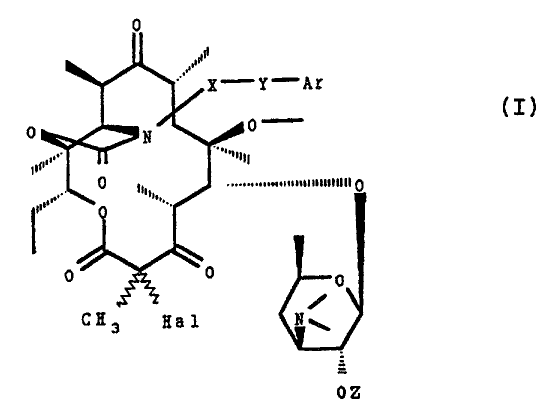 dans lesquels
- X représente un radical (NH)a, CH₂ ou SO₂ ou un atome d'oxygène, a représente le nombre 0 ou 1,
- Y représente un radical (CH₂)m-(CH=CH)n-(CH₂)o avec m+n+o ≤ 8, n = 0 ou 1,
- Ar représentant un radical aryle ou hétéroaryle, éventuellement substitué
- Hal représente un atome d'halogène
- Z représentant un atome d'hydrogène ou le reste d'un acide ainsi que leurs sels d'addition avec les acides.

2. Les composés de formule (I) tels que définis à la revendication 1 dans lesquels Z représente un atome d'hydrogène.

3. Les composés de formule (I) tels que définis à la revendication 1 ou 2 dans lesquels X représente un radical (NH)a, a conservant sa signification précédente.

4. Les composés de formule (I) tels que définis à la revendication 1, 2 ou 3 dans lesquels Hal représente un atome de fluor.

5. Les composés de formule (I) tels que définis à l'une quelconque les revendications 1 à 4 dans lesquels Y représente (CH₂)₃, (CH₂)₄ ou (CH₂)₅.

6. Les composés de formule (I) tels que définis à l'une quelconque les revendications 1 à 5 lesquels Ar représente un radical aryle hétérocyclique.

7. Les composés de formule (I) tels que définis à l'une quelconque les revendications 1 à 6 dans lesquels Ar représente un radical

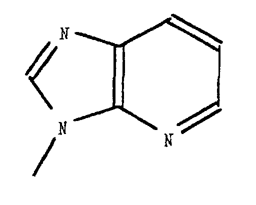

8. Les composés de formule (I) tels que définis à l'une quelconque des revendications 1 à 6 dans lesquels Ar représente un radical 4-quinoléinyle éventuellement mono ou polysubstitué sur l'un et/ou l'autre des 2 cycles de la quinoléine.

9. Les composés de formule (I) tels que définis à la revendication 8 dans lesquels Ar représente un radical 4-quinoléinyle non substitué.

10. Le composé de formule (I) tel que défini à la revendication 1 dont le nom suit :
- 11,12-didéoxy 3-de((2,6-didéoxy-3-C-méthyl-3-O-méthyl-alpha-L-ribohexopyranosyl) oxy)-2-fluoro-6-O-méthyl-3-oxo-12,11-(oxycarbonyl((4-(3H-imidazo(4,5-b) pyridin-3-yl) butyl) imino)) érythromycine A.

11. A titre de médicaments, les composés de formule (I) tels que définis à l'une quelconque des revendications 1 à 9, ainsi que leurs sels d'addition avec les acides pharmaceutiquement acceptables.

12. A titre de médicament, le composé de la revendication 10 ainsi que ses sels d'addition avec les acides pharmaceutiquement acceptables.

13. Les compositions pharmaceutiques renferment comme principe actif au moins un médicament défini à la revendication 11 ou 12.

14. Procédé de préparation des composés de formule (I) tels que définis à l'une quelconque des revendications 1 à 10, **caractérisé en ce que** l'on soumet un composé de formule (II) : 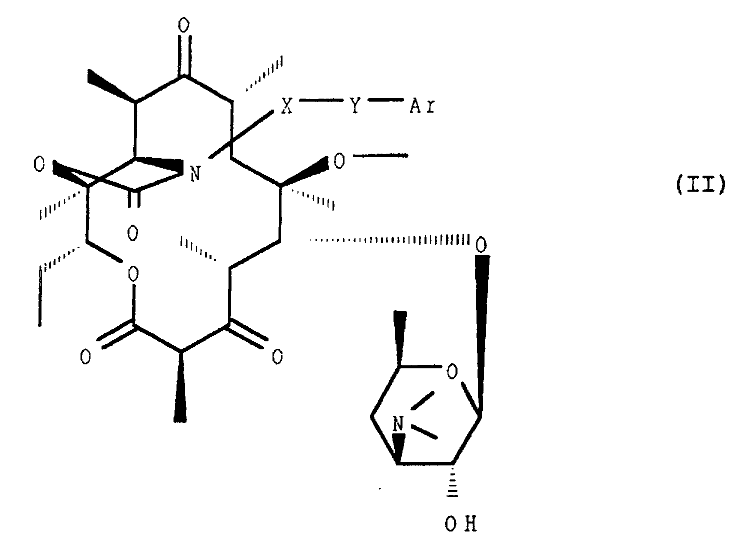 dans lesquels X, Y et Ar conservent leur signification précédente, à l'action d'un composé de formule (III) : 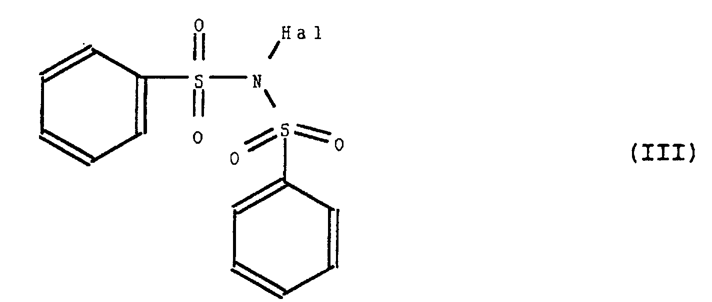 dans lequel Hal conserve sa signification précédente, pour obtenir le composé de formule (I) correspondant que l'on soumet si désiré à l'action d'un acide pour en former le sel.

## Claims

1. The compounds of formula (I): 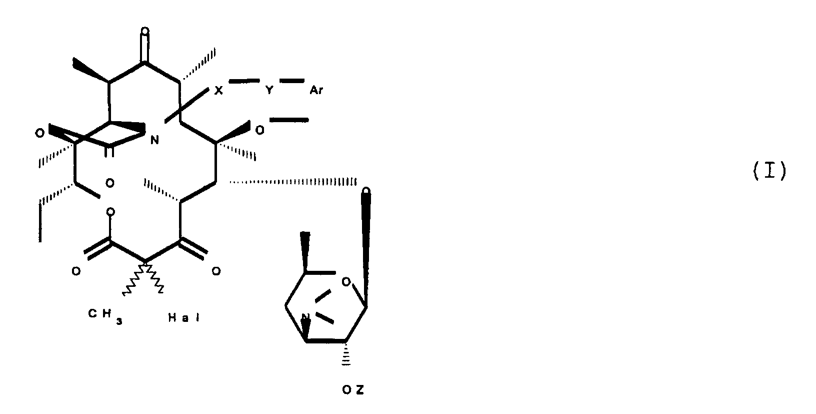 in which
- X represents an (NH)a, CH₂ or SO₂ radical or an oxygen atom, a represents the number 0 or 1,
- Y represents a (CH₂)m-(CH=CH)n-(CH₂)o radical with m+n+o ≤ 8, n = 0 or 1,
- Ar representing an optionally substituted aryl or heteroaryl radical,
- Hal represents a halogen atom
- Z representing a hydrogen atom or the remainder of an acid as well as their addition salts with acids.

2. The compounds of formula (I) as defined in claim 1 in which Z represents a hydrogen atom.

3. The compounds of formula (I) as defined in claim 1 or 2 in which X represents an (NH)a radical, a retaining its previous meaning.

4. The compounds of formula (I) as defined in claim 1, 2 or 3 in which Hal represents a fluorine atom.

5. The compounds of formula (I) as defined in any one of claims 1 to 4 in which Y represents (CH₂)₃, (CH₂)₄ or (CH₂)₅.

6. The compounds of formula (I) as defined in any one of claims 1 to 5 which Ar represents a heterocyclic aryl radical.

7. The compounds of formula (I) as defined in any one of claims 1 to 6 in which Ar represents a 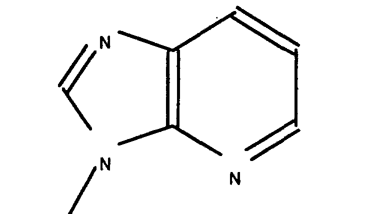 radical

8. The compounds of formula (I) as defined in any one of claims 1 to 6 in which Ar represents a 4-quinolinyl radical optionally mono- or polysubstituted on one and/or the other of the 2 quinoline rings.

9. The compounds of formula (I) as defined in claim 8 in which Ar represents a non-substituted 4-quinolinyl radical.

10. The compound of formula (I) as defined in claim 1 the name of which follows:
- 11,12-dideoxy 3-de((2,6-dideoxy-3-C-methyl-3-O-methyl-alpha-L-ribohexopyranosyl) oxy)-2-fluoro-6-O-methyl-3-oxo-12,11-(oxycarbonyl((4-(3H-imidazo(4,5-b) pyridin-3-yl) butyl) imino)) erythromycin A.

11. As medicaments, the compounds of formula (I) as defined in any one of claims 1 to 9, as well as their addition salts with pharmaceutically acceptable acids.

12. As a medicament, the compounds of claim 10 as well as their addition salts with pharmaceutically acceptable acids.

13. The pharmaceutical compositions containing at least one medicament defined in claim 11 or 12 as active ingredient.

14. Process for the preparation of the compounds of formula (I) as defined in any one of claims 1 to 10, **characterized in that** a compound of formula (II): 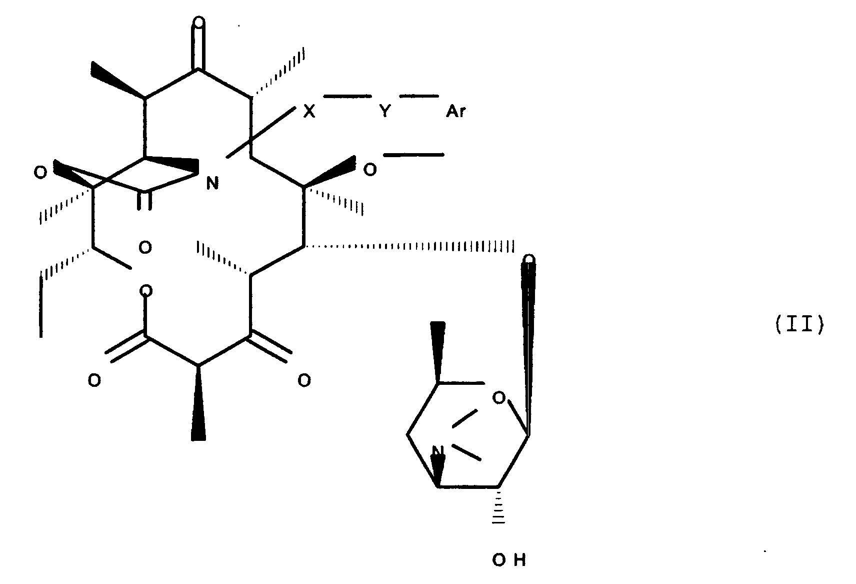 in which X, Y and Ar retain their previous meaning, is subjected to the action of a compound of formula (III): 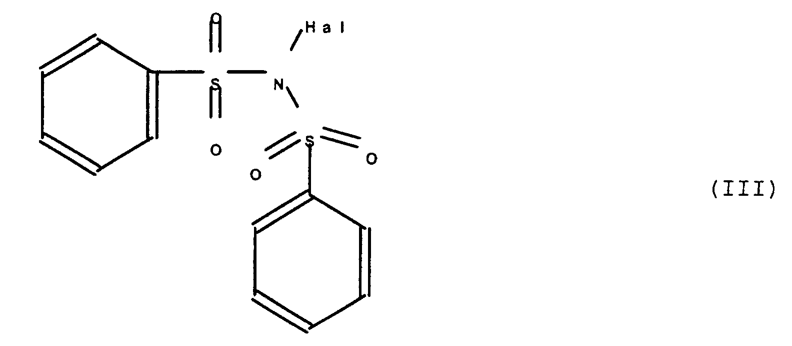 in which Hal retains its previous meaning, in order to obtain the corresponding compound of formula (I) which is subjected if desired to the action of an acid in order to form the salt.

## Patentansprüche

1. Verbindungen der Formel (I): 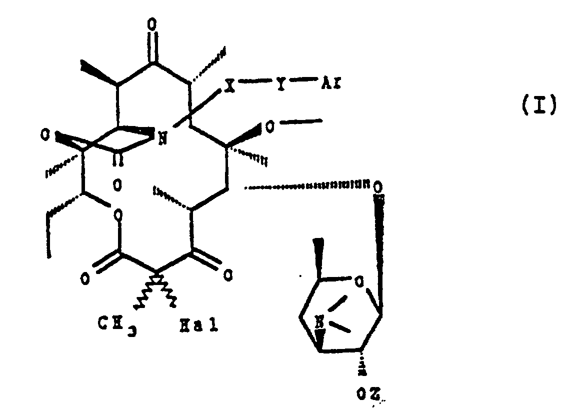 worin
- X eine Gruppe (NH)a, CH₂ oder SO₂ oder ein Sauerstoffatom darstellt; a die Zahl 0 oder 1 darstellt;
- Y eine Gruppe (CH₂)ₘ-(CH=CH)ₙ-(CH₂)o mit m+n+o ≤ 8, n = 0 oder 1 darstellt;
- Ar eine Aryl- oder Heteroaryl-Gruppe, die gegebenenfalls substituiert ist, darstellt;
- Hal ein Halogenatom darstellt;
- Z ein Wasserstoffatom oder den Rest einer Säure darstellt;
sowie ihre Additionssalze mit Säuren.

2. Verbindungen der Formel (I) nach Anspruch 1, worin Z ein Wasserstoffatom darstellt.

3. Verbindungen der Formel (I) nach Anspruch 1 oder 2, worin X eine Gruppe (NH)a darstellt, worin a seine vorstehende Bedeutung behält.

4. Verbindungen der Formel (I) nach Anspruch 1, 2 oder 3, worin Hal ein Fluoratom darstellt.

5. Verbindungen der Formel (I) nach einem der Ansprüche 1 bis 4, worin Y (CH₂)₃, (CH₂)₄ oder (CH₂)₅ darstellt.

6. Verbindungen der Formel (I) nach einem der Ansprüche 1 bis 5, worin Ar eine heterocyclische Aryl-Gruppe darstellt.

7. Verbindungen der Formel (I) nach einem der Ansprüche 1 bis 6, worin Ar eine Gruppe 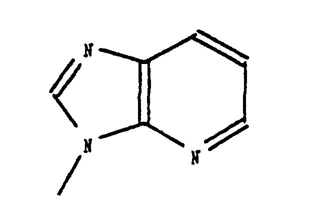 darstellt.

8. Verbindungen der Formel (I) nach einem der Ansprüche 1 bis 6, worin Ar eine 4-Chinolinyl-Gruppe darstellt, die gegebenenfalls an dem einen und/oder dem anderen der zwei Ringe des Chinolins mono- oder polysubstituiert ist.

9. Verbindungen der Formel (I) nach Anspruch 8, worin Ar eine nicht-substituierte 4-Chinolinyl-Gruppe darstellt.

10. Verbindung der Formel (I) nach Anspruch 1 mit dem folgenden Namen:
- 11,12-Didesoxy-3-de((2,6-didesoxy-3-C-methyl-3-O-methyl-alpha-L-ribohexapyranosyl)oxy)-2-fluor-6-Omethyl-3-oxo-12,11-(oxycarbonyl((4-(3H-imidazo(4,5-b)-pyridin-3-yl)butyl)imino))erythromycin A.

11. Verbindungen der Formel (I) nach einem der Ansprüche 1 bis 9, sowie ihre Additionssalze mit pharmazeutisch annehmbaren Säuren als Arzneimittel.

12. Verbindung nach Anspruch 10 sowie ihre Additionssalze mit pharmazeutisch annehmbaren Säuren als Arzneimittel.

13. Pharmazeutische Zusammensetzungen, die als Wirksubstanz mindestens ein Arzneimittel nach Anspruch 11 oder 12 umfassen.

14. Verfahren zur Herstellung der Verbindungen der Formel (I) nach einem der Ansprüche 1 bis 10, **dadurch gekennzeichnet, daß** man eine Verbindung der Formel (II) : 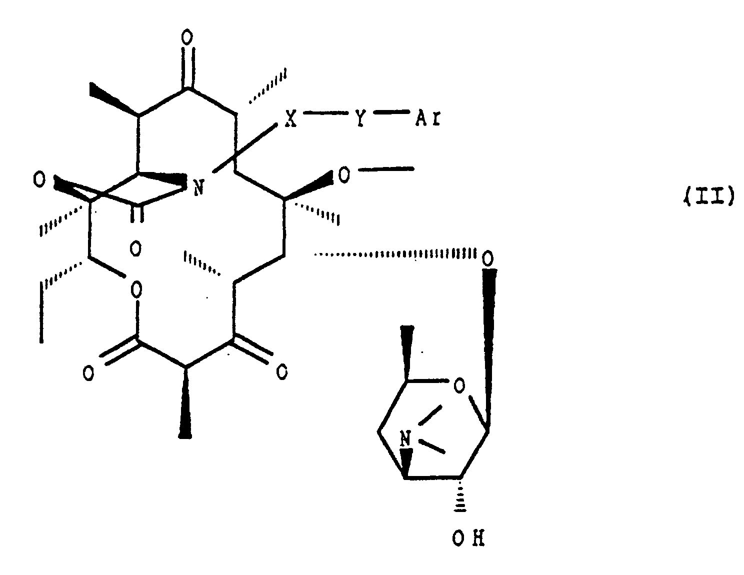 worin X, Y und Ar ihre vorstehende Bedeutung behalten, der Wirkung einer Verbindung der Formel (III): 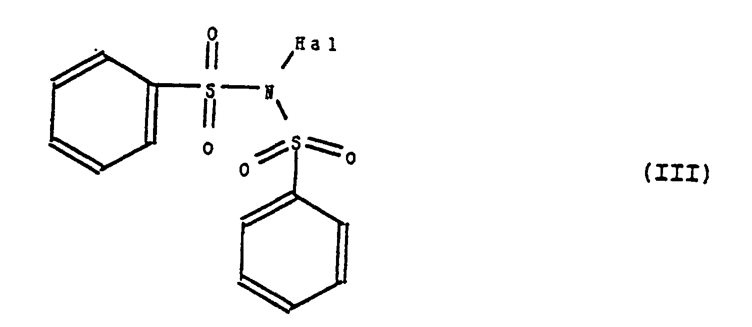 worin Hal seine vorstehende Bedeutung behält, unterwirft, um die entsprechende Verbindung der Formel (I) zu erhalten, die man, wenn gewünscht, der Wirkung einer Säure unterwirft, um daraus das Salz zu bilden.
